# EUROPÄISCHE PATENTSCHRIFT

(11) **EP 3 697 465 B1**
(45) Veröffentlichungstag und Bekanntmachung des Hinweises auf die Patenterteilung: **18.03.2026**
(21) Anmeldenummer: 18788740.1
(22) Anmeldetag: 15.10.2018
(51) Int. Cl.: A61M 1/14, A61M 1/36, A61M 1/16

(54) **SCHALTUNGSANORDNUNG ZUR SCHUTZLEITERANBINDUNG AN MINDESTENS ZWEI FLÜSSIGKEITSFÜHRENDE LEITUNGEN UND VERFAHREN ZUR ÜBERPRÜFUNG EINER SCHUTZLEITERANBINDUNG**
CIRCUIT FOR CONNECTING A PROTECTIVE CONDUCTOR TO AT LEAST TWO LIQUID-CONVEYING LINES AND METHOD FOR CHECKING A PROTECTIVE CONDUCTOR CONNECTION
ENSEMBLE CIRCUIT DESTINÉ À ÊTRE RELIÉ PAR CONDUCTEURS DE PROTECTION À AU MOINS DEUX CONDUITES À CIRCULATION DE LIQUIDE ET PROCÉDÉ DESTINÉ À CONTRÔLER UNE LIAISON PAR CONDUCTEURS DE PROTECTION

(30) Priorität: 19.10.2017 DE 102017009752
(43) Veröffentlichungstag der Anmeldung: 26.08.2020
(73) Patentinhaber: Fresenius Medical Care Deutschland GmbH, 61352 Bad Homburg v.d.H (DE)
(72) Erfinder: DANZ, Tim, 97717 Wirmsthal (DE); SCHMITT, Thomas, 97456 Dittelbrunn (DE); BREITKOPF, Berthold, 97424 Schweinfurt (DE); ASCHENBRENNER, Paul, 97453 Schonungen (DE); SCHEURING, Oswald, 97532 Üchtelhausen (DE)
(74) Vertreter: Oppermann, Frank
(86) Internationale Anmeldenummer: PCT/EP2018/078076
(87) Internationale Veröffentlichungsnummer: WO 2019/076814

(56) Entgegenhaltungen:
- EP-A1- 0 846 470
- EP-A2- 1 623 733
- WO-A1-2004/108206
- DE-A1- 102013 107 323
- US-A1- 2009 210 162

## Beschreibung

Die Erfindung betrifft eine Schaltungsanordnung zur Schutzleiteranbindung an mindestens zwei flüssigkeitsführende Leitungen, insbesondere an mindestens zwei flüssigkeitsführende Leitungen, die von dem Inneren eines Gehäuses eines medizintechnischen Gerätes, insbesondere des Gehäuses eines Blutbehandlungsgerätes, nach Außen geführt sind. Darüber hinaus betrifft die Erfindung ein medizintechnisches Gerät mit einem Gehäuse und mindestens zwei flüssigkeitsführenden Leitungen und einer derartigen Schaltungsanordnung sowie ein Verfahren zur Überprüfung der Schutzleiteranbindung an mindestens zwei flüssigkeitsführende Leitungen, die von dem Inneren eines Gehäuses eines medizintechnischen Gerätes, insbesondere des Gehäuses eines Blutbehandlungsgerätes, nach Außen geführt sind.

Die medizintechnischen Geräte, insbesondere Blutbehandlungsgeräte, erfordern besondere elektrische Schutzmaßnahmen, um den elektrischen Sicherheitsvorschriften zu genügen. Für die Stromversorgung verfügen die medizintechnische Geräte über ein Netzteil, das einen zentralen Schutzleiteranschluss aufweist, der mit dem Schutzleitersystem der festen Elektroinstallation verbunden wird, das sich auf Erdpotential befindet. Die sicherheitstechnische Kontrolle (STK) medizintechnischer Geräte schließt die Prüfung des elektrischen Widerstands der Schutzleiteranbindung ein.

Eine Schutzmaßnahme zur Erfüllung der elektrischen Sicherheitsvorschriften (IEC 60601-1) ist bei Dialysegeräten die Anbringung einer elektrischen Schutz-Vorrichtung an den flüssigkeitsführenden Leitungen des Flüssigkeitsteils, um eine elektrische Verbindung zu den in den Flüssigkeitsleitungen befindlichen Flüssigkeiten herzustellen. Diese Schutz-Vorrichtung kann als rohrförmiger Körper aus einem elektrisch leitenden Material ausgebildet sein, an den die Flüssigkeitsleitung angeschlossen ist. Der rohrförmige Körper, der auch als Tülle bezeichnet wird, ist über eine Verbindungsleitung mit einem Schutzleiter oder einem Potentialausgleich verbunden. Die Tülle kann auch in eine Ausnehmung des Gehäuses eingesetzt sein, um eine Flüssigkeitsleitung aus dem Gehäuse herauszuführen. Bei den bekannten Dialysegeräten ist einer Flüssigkeitsleitung nur eine einzige derartige Schutz-Vorrichtung zur Kontaktierung zugeordnet.

Die bekannten Blutbehandlungsgeräte, insbesondere Dialysegeräte, verfügen über einen extrakorporalen Blutkreislauf und einen Dialysierflüssigkeitsteil oder Dialysierflüssigkeitkreis. Der extrakorporale Blutkreislauf umfasst die Blutkammer und der Dialysierflüssigkeitsteil die Dialysierflüssigkeitskammer eines Dialysators (Filter), der durch eine semipermeable Membran in die beiden Kammern unterteilt ist. Der Dialysator ist im Allgemeinen eine austauschbare Einheit, die an der Außenseite des Gehäuses des Dialysegerätes leicht zugänglich befestigt ist.

Die Dialysegeräte können über eine Einrichtung zur Herstellung von Dialysierflüssigkeit aus Frischwasser und Konzentraten verfügen, die sich innerhalb des Gehäuses befindet. Die frische Dialysierflüssigkeit wird der Dialysierflüssigkeitskammer von der Einrichtung zur Herstellung von Dialysierflüssigkeit über eine Dialysierflüssigkeitszuführleitung zugeführt und aus der Dialysierflüssigkeitskammer wird gebrauchte Dialysierflüssigkeit über eine Dialysierflüssigkeitsabführleitung abgeführt. Da sich der Dialysator nicht in dem Gehäuse des Dialysegerätes befindet, müssen die Dialysierflüssigkeitsleitungen von dem Inneren des Gehäuses nach Außen geführt werden.

Die EP 0 846 470 A1 beschreibt eine Schaltungsanordnung zur Schutzleiteranbindung an mindestens zwei flüssigkeitsführende Leitungen, wobei die Schaltungsanordnung eine Mehrzahl von Schutz-Vorrichtungen zur elektrischen Kontaktierung aufweist, die derart ausgebildet sind, dass mit jeder Schutz-Vorrichtung eine elektrische Verbindung zu einer in der Leitung befindlichen Flüssigkeit herstellbar ist. Die Lehre der EP 0 846 470 A1 liegt daher darin, jeder flüssigkeitsführenden Leitung nur eine einzige Schutz-Vorrichtung zuzuordnen.

Der Erfindung liegt die Aufgabe zugrunde, ein verbessertes Schutzleiterkonzept anzugeben. Der Erfindung liegt insbesondere die Aufgabe zugrunde, eine Schaltungsanordnung bereitzustellen, mit der sich mit einem verhältnismäßig geringen technischen Aufwand die elektrische Sicherheit von medizintechnischen Geräten, insbesondere Dialysegeräten, verbessern lässt. Darüber hinaus ist eine Aufgabe der Erfindung, ein medizintechnisches Gerät mit einer verbesserten Schutzleiteranbindung zu schaffen. Eine Aufgabe der Erfindung ist auch, die Überprüfung der Schutzleiteranbindung zu vereinfachen, und ein Verfahren anzugeben, das die Überprüfung der Schutzleiteranbindung mit der erfindungsgemäßen Schaltungsanordnung auf einfache Weise erlaubt.

Die Lösung dieser Aufgaben erfolgt erfindungsgemäß mit den Merkmalen der unabhängigen Patentansprüche. Die abhängigen Ansprüche betreffen vorteilhafte Ausführungsformen der Erfindung.

Die erfindungsgemäße Schaltungsanordnung weist eine Mehrzahl von Schutz-Vorrichtungen zur elektrischen Kontaktierung auf, die derart ausgebildet sind, dass mit jeder Schutz-Vorrichtung zur elektrischen Kontaktierung eine elektrische Verbindung zu einer in der Leitung befindlichen Flüssigkeit herstellbar ist. Derartige Schutz-Vorrichtungen zur elektrischen Kontaktierung können beispielsweise als rohrförmige Körper ausgebildet sein, an dessen Endstücke die flüssigkeitsführenden Leitungen angeschlossen werden können. Die Endstücke können als Anschlussstücke ausgebildet sein, an denen Schlauchleitungen angeschlossen werden können. Beispielsweise können die Schlauchleitungen einfach auf die Endstücke aufgeschoben werden. Es ist aber auch möglich, dass die Endstücke über Stecker oder Buchsen oder andere Konnektoren verfügen. Die Schutz-Vorrichtungen können auch als Gehäusedurchführung ausgebildet sein. Die elektrische Verbindung zu der in der Flüssigkeitsleitung befindlichen Flüssigkeit kann dadurch hergestellt werden, dass die Schutz-Vorrichtungen aus einem elektrisch leitfähigen Material, insbesondere Metall, bestehen. Die Schutz-Vorrichtungen können aber auch aus einem nicht-elektrisch leitenden Material bestehen, wenn sie über elektrisch leitende mit der Flüssigkeit in Kontakt stehende Einsatzstücke, insbesondere Metallstücke, verfügen.

Die erfindungsgemäße Schaltungsanordnung zeichnet sich dadurch aus, dass jeder flüssigkeitsführenden Leitung mindestens zwei Schutz-Vorrichtungen zur elektrischen Kontaktierung zugeordnet sind. Folglich werden für jede flüssigkeitsführende Leitung zwei Schutzmaßnahmen (MOP: "Means of Protection) getroffen. Dadurch wird die elektrische Sicherheit verbessert, ohne dass andere Maßnahmen, beispielsweise weitere konstruktive Maßnahmen zur Vergrößerung von Luft- oder Kriechstrecken und/oder für eine verbesserte elektrische Isolation, erforderlich wären. Die Sicherheit für einen an die flüssigkeitsführenden Leitungen angeschlossenen Patienten wird insgesamt erhöht, da die Leitungen mit zwei voneinander unabhängigen Maßnahmen mit einem Schutzleiter oder einem Schutzpotential verbunden sind.

Die erfindungsgemäße Schaltungsanordnung zeichnet sich weiterhin dadurch aus, dass die mindestens zwei Schutz-Vorrichtungen zur elektrischen Kontaktierung einer flüssigkeitsführenden Leitung jeweils mit einer anderen Schutz-Vorrichtung zur elektrischen Kontaktierung einer anderen flüssigkeitsführenden Leitung elektrisch verbunden sind. Folglich ist eine der mindestens zwei Schutz-Vorrichtungen zur elektrischen Kontaktierung, die der einen flüssigkeitsführenden Leitung zugeordnet sind, mit einer der mindestens zwei Schutz-Vorrichtungen zur elektrischen Kontaktierung, die der anderen flüssigkeitsführenden Leitung zugeordnet sind, elektrisch verbunden, während eine andere der mindestens zwei Schutz-Vorrichtungen zur elektrischen Kontaktierung, die der einen flüssigkeitsführenden Leitung zugeordnet sind, mit einer anderen der mindestens zwei Schutz-Vorrichtungen zur elektrischen Kontaktierung, die der anderen flüssigkeitsführenden Leitung zugeordnet sind, elektrisch verbunden ist.

Der Vorteil der elektrischen Verbindung der den beiden flüssigkeitsführenden Leitungen zugeordneten Schutzvorrichtungen untereinander hat den Vorteil, dass bei Ausfall der Schutzleiteranbindung einer flüssigkeitsführenden Leitung, beispielsweise infolge einer Leiterunterbrechung, diese Leitung noch durch die Schutzleiteranbindung der anderen flüssigkeitsführenden Leitung an den Schutzleiter oder Potentialausgleich angebunden ist. Die Erhöhung der Redundanz führt zu einer erhöhten Sicherheit für den Patienten.

Weitere Vorteile der erfindungsgemäßen Schaltungsanordnung kommen dann zum tragen, wenn die Flüssigkeitsleitungen aus dem Inneren des Gehäuses nach Außen herausgeführt sind, d.h. die Gehäusewand durchdringen. Dann ist von den mindestens zwei Schutz-Vorrichtungen zur elektrischen Kontaktierung, die jeder flüssigkeitsführenden Leitung zugeordnet sind, mindestens eine Schutz-Vorrichtung zur elektrischen Kontaktierung einem im Inneren des Gehäuses liegenden Leitungsabschnitt der flüssigkeitsführenden Leitung und mindestens eine Schutz-Vorrichtung zur elektrischen Kontaktierung einem zumindest teilweise außerhalb des Gehäuses liegenden Leitungsabschnitt der flüssigkeitsführenden Leitung zugeordnet. Eine bevorzugte Ausführungsform sieht vor, dass eine Schutz-Vorrichtung zur elektrischen Kontaktierung einer flüssigkeitsführenden Leitung, die dem im Inneren des Gehäuses liegenden Leitungsabschnitt der einen flüssigkeitsführenden Leitung zugeordnet ist, mit einer Schutz-Vorrichtung zur elektrischen Kontaktierung einer anderen flüssigkeitsführenden Leitung, die dem zumindest teilweise außerhalb des Gehäuses liegenden Leitungsabschnitt der anderen flüssigkeitsführenden Leitung zugeordnet ist, elektrisch verbunden ist. Die dem im Inneren des Gehäuses liegenden Leitungsabschnitt der flüssigkeitsführenden Leitung zugeordneten Schutz-Vorrichtungen sind jeweils mit einer elektrischen Verbindungsleitung an einen Schutzleiter oder einen Potentialausgleich angeschlossen.

Zumindest teilweise außerhalb des Gehäuses liegt ein Leitungsabschnitt in Bezug auf die Position der ihr zugeordneten Schutz-Vorrichtung, wenn sich die Schutz-Vorrichtung zu einem Teil innerhalb des Gehäuses und zum anderen Teil außerhalb des Gehäuses befindet. Dies ist insbesondere dann der Fall, wenn die Schutzvorrichtung als Gehäusedurchführung in der Gehäusewand montiert ist und die elektrische Kontaktierung der Schutz-Vorrichtung vorzugsweise an den in das Gehäuseinnere weisenden Teil der Schutz-Vorrichtung erfolgt. In dieser Ausführungsform werden Kabelverbindungen, die das Gehäuse durchdringen, vermieden. Eine als Gehäusedurchführung ausgeführte Schutz-Vorrichtung kann elektrisch isoliert gegenüber dem Gehäuse montiert werden, beispielsweise durch umlaufende Isolatoren.

Dieses Schutzleiterkonzept mit einer "kreuzweisen" Verschaltung der Schutz-Vorrichtungen erlaubt eine besonderes einfache Überprüfung sämtlicher Schutzleiteranbindungen einer flüssigkeitsführenden Leitung nur mit Widerstandsmessungen, die ohne ein Öffnen des Gehäuses durchführbar sind, da zwei unabhängige Messpunkte an den von Außen zugänglichen Schutz-Vorrichtungen geschaffen werden, die dem außerhalb des Gehäuses liegenden Leitungsabschnitt der flüssigkeitsführenden Leitung zugeordnet sind. Darüber hinaus wird mit diesem Schutzleiterkonzept eine Erdung der Leitungen auch dann sichergestellt, wenn eine der elektrischen Verbindungsleitungen an den Schutzleiter oder Potentialausgleich unterbrochen sein sollte. Die Erdung erfolgt dann über die andere Verbindungsleitung. Somit kann mit einer Messung ermittelt werden, ob alle flüssigkeitsführenden Leitungen über zumindest eine Schutz-Vorrichtung mit dem Schutzleiter verbunden sind.

Das erfindungsgemäße Verfahren zur Überprüfung einer Schutzleiteranbindung an mindestens zwei flüssigkeitsführende Leitungen, die von dem Inneren eines Gehäuses eines medizintechnischen Gerätes nach Außen geführt sind, mit der erfindungsgemäßen Schaltungsanordnung sieht vor, dass der elektrische Widerstand zwischen den Schutz-Vorrichtungen zur elektrischen Kontaktierung, die dem außerhalb des Gehäuses liegenden Leitungsabschnitten der flüssigkeitsführenden Leitungen zugeordnet sind, und dem Schutzleiter oder Potentialausgleich gemessen wird.

Bei einer Ausführungsform besteht das Gehäuse des medizintechnischen Gerätes aus einem elektrisch leitenden Material, wobei der Schutzleiter oder Potentialausgleich elektrisch mit dem Gehäuse verbunden ist. Wenn der Schutzleiter oder Potentialausgleich elektrisch mit einem elektrisch leitenden Gehäuse verbunden ist, kann der elektrische Widerstand zwischen den außenliegenden Schutz-Vorrichtungen zur elektrischen Kontaktierung und dem Gehäuse auf einfache Weise gemessen werden, ohne das Gehäuse öffnen zu müssen, da sämtliche Messpunkte von außen zugänglich sind.

Eine alternative Ausführungsform sieht ein Gehäuse des medizintechnischen Gerätes aus einem elektrisch nicht-leitenden Material vor, wobei der Schutzleiter oder Potentialausgleich elektrisch mit einer von Außen zugänglichen elektrischen Mess-Kontaktstelle an dem elektrisch nicht-leitenden Gehäuse verbunden ist. Bei dieser Ausführungsform kann der elektrische Widerstand zwischen den außenliegenden Schutz-Vorrichtungen und der Mess-Kontaktstelle einfach gemessen werden. Alternativ oder zusätzlich kann der elektrische Widerstand zwischen den außenliegenden Schutz-Vorrichtungen und dem Schutzkontakt im Netzstecker des medizintechnischen Gerätes gemessen werden.

Der Schutzleiter oder Potentialausgleich kann mit dem Schutzleitersystem einer festen Elektroinstallation verbunden sein.

Die mindestens zwei flüssigkeitsführenden Leitungen können Teil eines Flüssigkeitssystems des medizintechnischen Gerätes sein, das auch als Hydrauliksystem bezeichnet wird. In den flüssigkeitsführenden Leitungen kann sich eine medizinische Flüssigkeit, insbesondere Dialysat, befinden. Das medizintechnische Gerät kann eine erste flüssigkeitsführende Leitung und eine zweite flüssigkeitsführende Leitung aufweisen, wobei die beiden flüssigkeitsführenden Leitung einen Flüssigkeitskreis bilden, der eine Zulaufleitung und eine Rücklaufleitung umfassen kann. Der Flüssigkeitskreis kann dadurch geschaffen werden, dass die Enden der Flüssigkeitsleitungen miteinander verbunden werden. Die Verbindung kann mittels eines Kurschlussstücks oder eines Dialysators (Filters) erfolgen. Bei einem Dialysegerät kann das Flüssigkeitssystem die zu dem Dialysator (Filter) führende Dialysierflüssigkeitszuführleitung und die von dem Dialysator (Filter) abgehende Dialysierflüssigkeitsabführleitung umfassen.

Im Folgenden werden mehrere Ausführungsbeispiele der Erfindung unter Bezugnahme auf die Zeichnungen im Einzelnen beschrieben.

Es zeigen:
- Fig. 1: einen Teil des Gehäuses eines Dialysegerätes mit der erfindungsgemäßen Schaltungsanordnung in stark vereinfachter schematischer Darstellung,
- Fig. 2: eine Schutz-Vorrichtung zur Kontaktierung in vergrößerter Darstellung,
- Fig. 3: einen Ausschnitt aus dem Gehäuse mit einer Mess-Kontaktstelle einer alternativen Ausführungsform der erfindungsgemäßen Schaltungsanordnung,
- Fig. 4: eine schematische Darstellung einer weiteren Ausführungsform der erfindungsgemäßen Schaltungsanordnung,
- Fig. 5: eine weitere Ausführungsform der erfindungsgemäßen Schaltungsanordnung, bei der sämtliche Schutz-Vorrichtungen im Inneren des Gehäuses angeordnet sind,
- Fig. 6: eine weitere Ausführungsform der erfindungsgemäßen Schaltungsanordnung, bei der sämtliche Schutz-Vorrichtungen im Inneren des Gehäuses angeordnet sind, und
- Fig. 7: eine als Gehäusedurchführung ausgebildete Schutz-Vorrichtung in schematischer Darstellung.

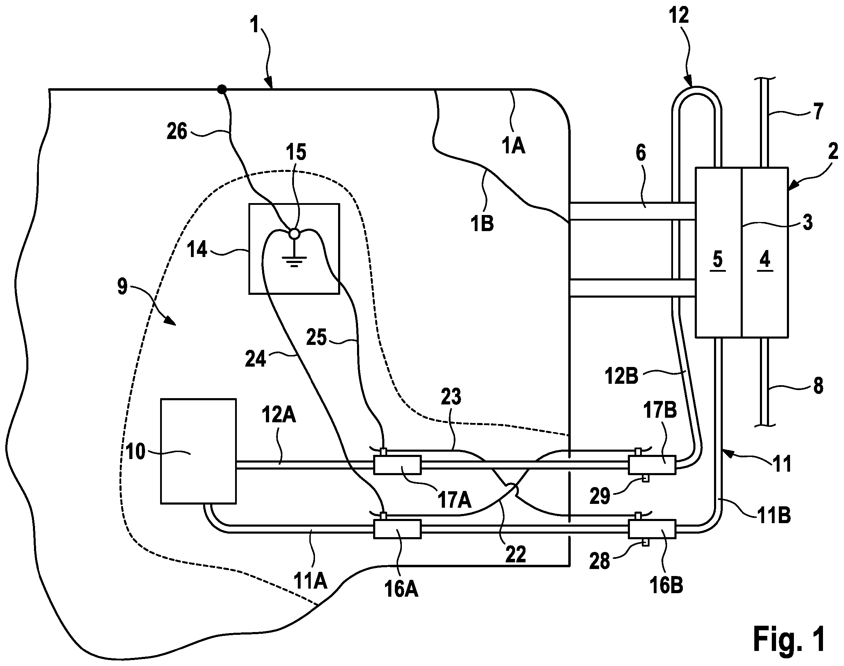

Fig. 1 zeigt die für die Erfindung wesentlichen Komponenten eines Blutbehandlungsgerätes als Beispiel für ein medizinisches Gerät in stark vereinfachter schematischer Darstellung. Das Blutbehandlungsgerät ist bei dem vorliegenden Ausführungsbeispiel ein Dialysegerät.

Das Dialysegerät weist ein Gehäuse 1 auf, das aus einem elektrisch leitfähigen Material besteht (Metallgehäuse). Das Gehäuse 1 kann aus mehreren Gehäuseteilen 1A, 1B bestehen. Darüber hinaus weist das Dialysegerät einen in Fig. 1 nur schematisch dargestellten Dialysator 2 auf, der durch eine semipermeable Membran 3 in eine Blutkammer 4 und eine Dialysierflüssigkeitskammer 5 unterteilt ist. Der Dialysator 2 kann an einer Halterung 6 befestigt werden, die an der Außenseite des Gehäuses 1 vorgesehen ist. Zu der Blutkammer 4 des Dialysators 2 führt eine Blutzuführleitung 7 und eine Blutabführleitung 8 geht von der Blutkammer 4 ab. Die Blutkammer 4 und die Blutzuführ- und Blutabführleitung 7, 8 bilden den extrakorporalen Blutkreislauf.

Das Dialysegerät verfügt über einen Dialysierflüssigkeitskreis 9, der eine Einrichtung 10 zur Herstellung von Dialysierflüssigkeit aus Frischwasser und Konzentraten aufweist. Von der Einrichtung 10 zur Herstellung von Dialysierflüssigkeit führt eine Dialysierflüssigkeitszuführleitung 11 zu einem Einlass der Dialysierflüssigkeitskammer 5 und von einem Auslass der Dialysierflüssigkeitskammer 5 führt eine Dialysierflüssigkeitsabführleitung 12 zu dieser Einrichtung 10 oder einen Auslauf.

Die Dialysierflüssigkeitszuführleitung 11 und die Dialysierflüssigkeitsabführleitung 12 durchdringen die Wand des Gehäuses 1 und haben jeweils einen im Inneren des Gehäuses liegenden Leitungsabschnitt 11A bzw. 12A, der nachfolgend als innerer Leitungsabschnitt bezeichnet wird, und einen außerhalb des Gehäuses liegenden Leitungsabschnitt 11B bzw. 12B, der nachfolgend als äußerer Leitungsabschnitt bezeichnet wird.

Darüber hinaus weist das Dialysegerät ein Netzteil 14 auf, das einen Schutzleiter oder einen Potentialausgleich 15 umfasst, der über den Schutzkontakt des Netzsteckers des Dialysegeräts an das Schutzleitersystem einer nicht dargestellten festen Elektroinstallation angeschlossen werden kann.

Das Flüssigkeitssystem des Dialysegerätes kann weitere Komponenten umfassen, die beispielsweise Aktoren oder Sensoren aufweisen.

Sämtliche technische Einrichtungen des Dialysegerätes erfordern besondere sicherheitstechnische Maßnahmen, die nachfolgend beschrieben werden.

Der Dialysierflüssigkeitszuführleitung 11 sind zwei Schutz-Vorrichtungen 16A, 16B zur elektrischen Kontaktierung zugeordnet, die derart ausgebildet sind, dass mit jeder Schutz-Vorrichtung eine elektrische Verbindung zu der in der Leitung 11 befindlichen Flüssigkeit, insbesondere zu dem zu der Dialysierflüssigkeitskammer 5 des Dialysators 2 fließenden Dialysats, herstellbar ist. Die eine innere Schutz-Vorrichtung 16A ist an dem inneren Leitungsabschnitt 11A angeordnet, während die andere äußere Schutz-Vorrichtung 16B an dem äußeren Leitungsabschnitt 11B angeordnet ist. Der Dialysierflüssigkeitsabführleitung 12 sind ebenfalls zwei Schutz-Vorrichtungen 17A, 17B zur elektrischen Kontaktierung zugeordnet, um eine elektrische Verbindung zu der in der Leitung 12 befindlichen Flüssigkeit, insbesondere zu dem von der Dialysierflüssigkeitskammer 5 des Dialysators 2 abfließenden Dialysat, herzustellen. Die eine innere Schutz-Vorrichtung 17A ist wieder an dem inneren Leitungsabschnitt 12A angeordnet, während die andere äußere Schutz-Vorrichtung 17B an dem äußeren Leitungsabschnitt 12B angeordnet ist. Die Dialysierflüssigkeitszuführ- und -abführleitung 11, 12 sind flexible Schlauchleitungen.

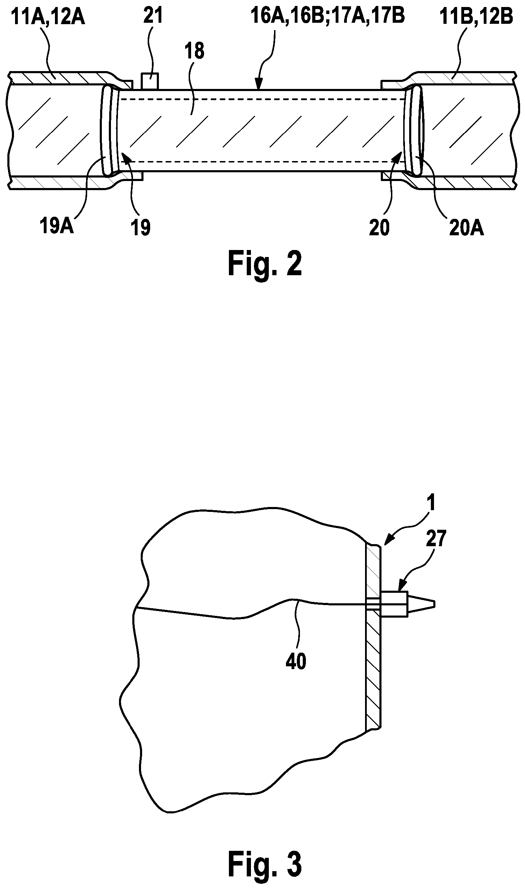

Fig. 2 zeigt eine der Schutz-Vorrichtungen 16A, 16B bzw. 17A, 17B (MOP: Means for Protection) zur elektrischen Kontaktierung in vergrößerter Darstellung. Die Schutz-Vorrichtung (MOP) weist einen rohrförmigen Körper 18 aus einem elektrisch leitenden Material auf. Beispielsweise kann die Schutz-Vorrichtung als eine Metalltülle ausgebildet sein. Die beiden Endstücke 19, 20 des rohrförmigen Körpers 18 weisen jeweils einen umlaufenden Wulst 19A, 20A auf. Die betreffenden Enden der Dialysierflüssigkeitszuführ- und -abführleitung 11, 12 sind auf die Endstücke 19, 20 des rohrförmigen Körpers 18 aufgeschoben. An dem rohrförmigen Körper 18 ist ein elektrischer Anschlussteil 21 vorgesehen, der dem Anschluss elektrischer Leitungen dient, um eine elektrische Verbindung zu dem rohrförmigen Körper und der durch den rohrförmigen Körper strömenden Flüssigkeit, insbesondere dem Dialysat, herzustellen. Anstelle von nur einem Anschlussteil, an den mehrerer Leitungen angeschlossen werden können, können an dem rohrförmigen Körper auch mehrere Anschlussteile vorgesehen sein, an die jeweils eine oder mehrere Leitungen angeschlossen werden können. In Fig. 1 ist der Anschluss der Verbindungsleitungen an eine Schutzvorrichtung nur mit einem Anschlussteil gezeigt.

Die beiden inneren und äußeren Schutz-Vorrichtungen 16A, 16B bzw. 17A, 17B der Dialysierflüssigkeitszuführ- und -abführleitung 11, 12 sind wie folgt verschaltet. Die innere Schutz-Vorrichtung 16A bzw. 17A einer Flüssigkeitsleitung 11 bzw. 12 ist mit der äußeren Schutz-Vorrichtung 16B bzw. 17B mindestens einer anderen Flüssigkeitsleitung 11 bzw. 12 elektrisch verbunden. Bei dem vorliegenden Ausführungsbeispiel ist die innere Schutz-Vorrichtung 16A der Dialysierflüssigkeitszuführleitung 11 über eine Verbindungsleitung 22 elektrisch mit der äußeren Schutz-Vorrichtung 17B der Dialysierflüssigkeitsabführleitung 12 elektrisch verbunden, während die innere Schutz-Vorrichtung 17A der Dialysierflüssigkeitsabführleitung 12 über eine Verbindungsleitung 23 elektrisch mit der äußeren Schutz-Vorrichtung 16B der Dialysierflüssigkeitszuführleitung 11 elektrisch verbunden ist. Darüber hinaus ist die innere Schutz-Vorrichtung 16A der Dialysierflüssigkeitszuführleitung 11 über eine Verbindungsleitung 24 an den Schutzleiter oder Potentialausgleich 15 angeschlossen, während die innere Schutz-Vorrichtung 17A der Dialysierflüssigkeitsabführleitung 12 über eine Verbindungsleitung 25 an den Schutzleiter oder Potentialausgleich 15 angeschlossen ist. Folglich sind die jeweils den einzelnen Flüssigkeitsleitungen 11, 12 zugeordneten Schutz-Vorrichtungen 16A, 16B bzw. 17A, 17B unabhängig voneinander niederohmig an die Erde angebunden. Die jeweiligen Verbindungsleitungen 22, 23, 24, 25 sind an den Anschlussteilen 21 der Schutz-Vorrichtungen angeschlossen. Die Schutz-Vorrichtungen 16A, 17A können für den Anschluss der betreffenden Leitungen 22 und 24 bzw. 23 und 25 aber auch jeweils über zwei getrennte Anschlussteile verfügen.

Eine Erdung sowohl der Dialysierflüssigkeitszuführleitung 11 als auch der Dialysierflüssigkeitsabführleitung 12 ist auch dann sichergestellt, wenn eine der beiden Leitungen 24 bzw. 25 zwischen den inneren Schutz-Vorrichtungen 16A bzw. 17A und dem Schutzleiter oder Potentialausgleich 15 unterbrochen sein sollte.

Der Schutzleiter oder Potentialausgleich 15 ist über eine weitere Verbindungsleitung 26 mit dem Gehäuse 1 verbunden, das bei dem vorliegenden Beispiel aus einem elektrisch leitenden Material besteht (Fig. 1). Wenn das Gehäuse 1 nicht aus einem elektrisch leitenden Material besteht, beispielsweise ein Kunststoffgehäuse ist, weist das Gehäuse eine von außen zugängliche Mess-Kontaktstelle 27 auf, die über eine Verbindungsleitung 40 mit dem Schutzleiter oder Potentialausgleich 15 verbunden ist. Fig. 3 zeigt diese Mess-Kontaktstelle 27 in schematischer Darstellung.

Ein PE-Leiter an einem metallischen Teil, das hier eine von einer leitfähigen Flüssigkeit, insbesondere Dialysat, durchflossenen Metalltülle ist, gilt im Sinne der Norm IEC 60601-1 als eine einzelne Schutzmaßnahme (MOP). Der weitere unabhängige PE-Leiter stellt im Sinne dieser Norm an dem gleichen Teil bei unabhängiger mechanischer Befestigung an diesem Teil eine weitere Schutzmaßnahme (MOP) dar. Die Anbindung von zwei unabhängigen Schutzleitern dient vor allem der Verbesserung der elektrischen Sicherheit insbesondere im Bereich der Hydraulik. Ein Vorteil des Sicherheitskonzepts liegt in der vereinfachten sicherheitstechnischen Kontrolle (STK).

Nachfolgend wird ein Verfahren zur Überprüfung einer Schutzleiteranbindung unter Verwendung der erfindungsgemäßen Schaltungsanordnung beschrieben. Zur sicherheitstechnischen Kontrolle (STK) wird mit einer ersten Messung der Widerstand zwischen der äußeren Schutz-Vorrichtung 16B der Dialysierflüssigkeitszuführleitung 11 und dem Gehäuse 1 (Fig. 1) bzw. der Mess-Kontaktstelle 27 des Gehäuses 1 (Fig. 3) und mit einer zweiten Messung wird der Widerstand zwischen der äußeren Schutz-Vorrichtung 17B der Dialysierflüssigkeitsabführleitung 12 und dem Gehäuse 1 bzw. der Mess-Kontaktstelle 27 des Gehäuses 1 gemessen. In Fig. 1 sind die unabhängigen Messpunkte an den Schutz-Vorrichtungen mit den Bezugszeichen 28, 29 bezeichnet. Die sicherheitstechnischen Kontrolle kann mit den im Feld üblicherweise benutzen Messmitteln erfolgen. Da sämtliche Messpunkte 28, 29 von außen zugänglich sind, braucht das Gehäuse nicht geöffnet zu werden. Eine Widerstandsmessung zwischen den Messpunkten 28, 29 erlaubt die Überprüfung der Verschaltung. Wenn der Widerstand zwischen den Messpunkten 28, 29 groß ist, kann darauf geschlossen werden, dass eine der Leitungen 22, 23, 24, 25 unterbrochen ist. Über eine zusätzliche Messung des elektrischen Widerstands zwischen den Messpunkten 28 bzw. 29 und dem Schutzkontakt im Netzstecker des Dialysegeräts kann ermittelt werden, ob eine ordnungsgemäße Verbindung zum Schutzleitersystem der Elektroinstallation herstellbar ist.

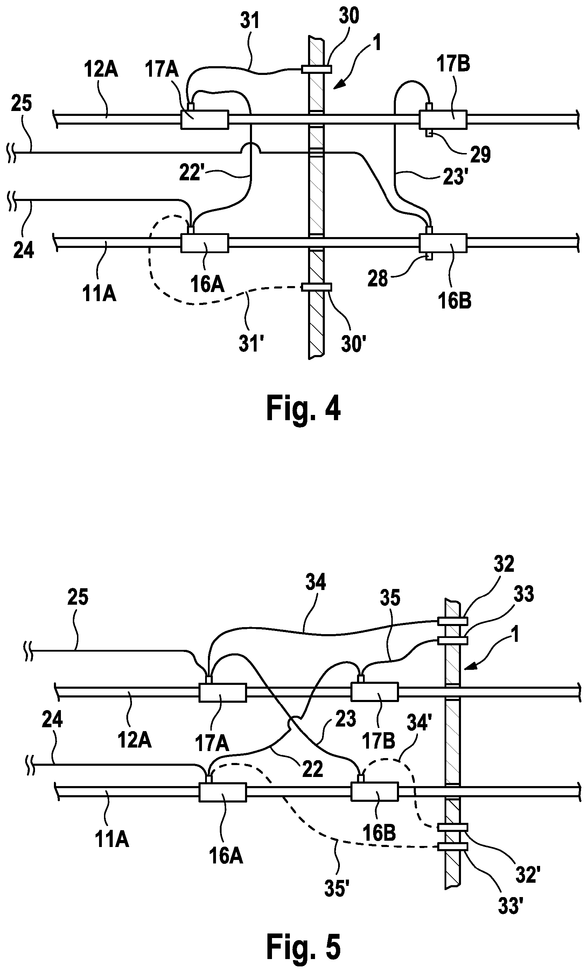

Fig. 4 zeigt in schematischer Darstellung eine weitere Ausführungsform der Schaltungsanordnung, die sich von der unter Bezugnahme auf die Figuren 1 bis 3 beschriebenen Schaltungsanordnung in der Verbindung der einzelnen Schutz-Vorrichtungen unterscheidet. Die einander entsprechenden Teile sind mit den gleichen Bezugszeichen versehen. Bei der alternativen Ausführungsform ist die innere Schutz-Vorrichtung 16A der einen flüssigkeitsführenden Leitung 11A über eine Verbindungsleitung 22' mit der inneren Schutz-Vorrichtung 17A der anderen flüssigkeitsführenden Leitung 12A verbunden, während die äußere Schutz-Vorrichtung 16B der einen flüssigkeitsführenden Leitung 11A über eine Verbindungsleitung 23' mit der äußeren Schutz-Vorrichtung 17B der anderen flüssigkeitsführenden Leitung 12A verbunden ist. Die inneren Schutz-Vorrichtungen 16A, 17A sind über eine Verbindungsleitung 24 mit einem in Fig. 4 nicht dargestellten ersten Sternpunkt elektrisch verbunden, der mit dem Schutzleiter verbunden ist, während die äußeren Schutz-Vorrichtungen 16B, 17B über eine Verbindungsleitung 25 mit einem nicht dargestellten zweiten Sternpunkt elektrisch verbunden sind, der mit dem Schutzleiter verbunden ist. Auch hier sind die flüssigkeitsführenden Leitungen bei Ausfall eines Schutzleiters, d.h. bei einer Unterbrechung einer der Leitungen 24, 25, die zu einem der beiden unabhängigen und mit dem Schutzleiter verbundenen Sternpunkten führen, immer noch durch den redundanten Sternpunktanschluss geerdet. Allerdings sind bei dieser Ausführungsform nicht sämtliche Messpunkte von außen zugänglich. Deshalb ist am Gehäuse 1 ein Messpunkt 30 vorgesehen, der über eine Leitung 31 mit der inneren Schutz-Vorrichtung 17A der einen flüssigkeitsführenden Leitung 12A elektrisch verbunden ist. Darüber hinaus kann ein weiterer Messpunkt 30' vorgesehen sein, der über eine Leitung 31' mit der inneren Schutz-Vorrichtung 16A der anderen flüssigkeitsführenden Leitung 11A elektrisch verbunden ist. Die Leitung 31' des alternativen oder optionalen Messpunktes 30' ist in gestrichelten Linien dargestellt. Mit einer Widerstandsmessung zwischen den beiden Messpunkten 30, 30' kann von außen der Widerstand zwischen den beiden inneren Schutz-Vorrichtungen 16A, 17A gemessen und ein möglicher Fehler eingegrenzt werden. Wenn beispielsweise der Widerstand zwischen den beiden Messpunkten 30, 30' niederohmig ist, der Widerstand zwischen dem Messpunkt 30 bzw. 30' und dem in Fig. 4 nicht dargestellten Schutzleiter aber hochohmig, kann auf einen Fehler in der elektrischen Verbindung der inneren Schutz-Vorrichtungen 16A, 17A und dem Schutzleiter geschlossen werden. Ein Fehler in der elektrischen Verbindung zwischen den inneren Schutz-Vorrichtungen 16A, 17A führt dazu, dass der Widerstand zwischen einem Messpunkt und dem Schutzleiter niederohmig und der Widerstand zwischen dem anderen Messpunkt und dem Schutzleiter hochohmig ist.

Fig. 5 zeigt in schematischer Darstellung eine weitere Ausführungsform der Schaltungsanordnung, die sich von der unter Bezugnahme auf die Figuren 1 bis 3 beschriebenen Schaltungsanordnung dadurch unterscheidet, dass sämtliche Schutz-Vorrichtungen 16A, 16B und 17A, 17B innerhalb des Gehäuses 1 angeordnet sind. Die einander entsprechenden Teile sind wieder mit den gleichen Bezugszeichen versehen. Bei dieser Ausführungsform sind am Gehäuse 1 zwei Messpunkte 32', 33 vorgesehen, die über Leitungen 34', 35 mit den Schutz-Vorrichtungen 16B, 17B der beiden flüssigkeitsführenden Leitungen 11A und 12A elektrisch verbunden sind. Darüber hinaus können zwei weitere Messpunkte 32 , 33' vorgesehen sein, die über Leitungen 34 , 35' mit den Schutz-Vorrichtungen 16A und 17A elektrisch verbunden sind. Die Leitungen 34 , 35' der optionalen Messpunkte 32 , 33' sind dargestellt. Durch Messen der Widerstände zwischen den Messpunkten 32, 33 und dem Schutzleiter kann überprüft werden, ob die Erdung ordnungsgemäß ist. Wenn vier Messpunkte 32, 33 und 32', 33' vorgesehen sind, kann auch der elektrische Widerstand zwischen den Schutz-Vorrichtungen 17A und 16B bzw. den Schutz-Vorrichtungen 16A und 17B gemessen werden, und ein möglicher Fehler eingegrenzt werden, ohne das Gehäuse zu öffnen.

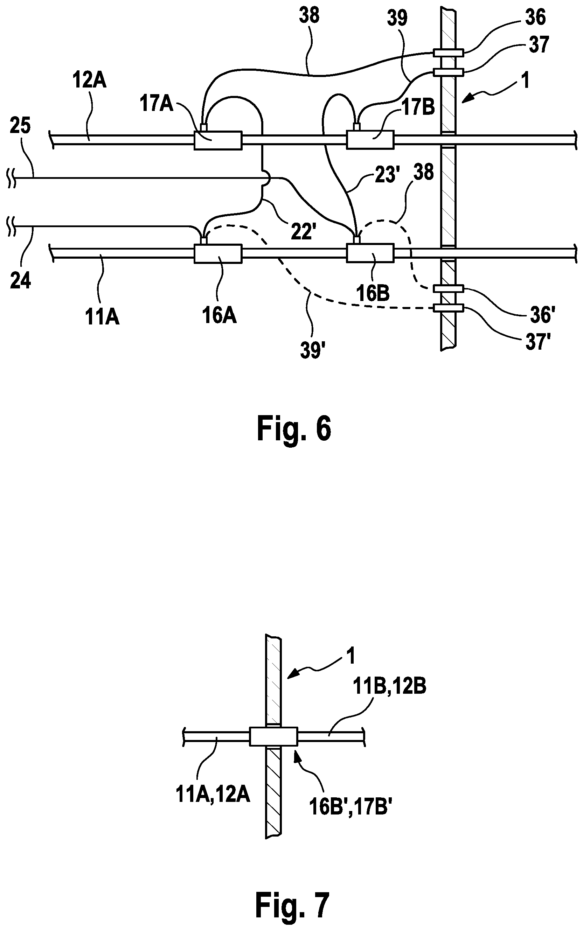

Fig. 6 zeigt in schematischer Darstellung eine weitere Ausführungsform der Schaltungsanordnung, die sich von der unter Bezugnahme auf Fig. 4 beschriebenen Schaltungsanordnung dadurch unterscheidet, dass sämtliche Schutz-Vorrichtungen 16A, 16B und 17A, 17B innerhalb des Gehäuses 1 angeordnet sind. Die einander entsprechenden Teile sind wieder mit den gleichen Bezugszeichen versehen. Bei dieser Ausführungsform sind am Gehäuse 1 zwei Messpunkte 36, 37 vorgesehen, die über Leitungen 38, 39 mit den Schutz-Vorrichtungen 17A, 17B einer der beiden flüssigkeitsführenden Leitungen 12A elektrisch verbunden sind. Darüber hinaus können zwei weitere Messpunkte 36', 37' vorgesehen sein, die über Leitungen 38', 39' mit den Schutz-Vorrichtungen 16A, 16B der anderen flüssigkeitsführenden Leitung 11A elektrisch verbunden sind. Die Leitungen 38', 39' der alternativen oder optionalen Messpunkte 36', 37' sind in gestrichelten Linien dargestellt. Die Überprüfung der Erdung kann mit den nach außen geführten Messpunkten wieder durch die oben beschriebenen Widerstandsmessungen erfolgen.

Fig. 7 zeigt in stark vereinfachter schematischer Darstellung eine Schutz-Vorrichtung, die als eine Gehäusedurchführung ausgebildet ist. Diese Schutz-Vorrichtung 16B', 17B' kann eine der beiden Schutz-Vorrichtungen 16B oder 17B sein, die in den Figuren 1 bis 6 gezeigt sind. Bei dieser Ausführungsform kann das Gehäuse 1 aus einem elektrisch leitenden Material bestehen. Wenn die Schutz-Vorrichtung einen Körper aus einem elektrisch leitenden Material aufweist, durch den Flüssigkeit strömt, ist dieser Körper vorzugsweise gegenüber dem elektrisch leitenden Gehäuse elektrisch isoliert.

Bei den oben beschriebenen Ausführungsformen können die einzelnen Messpunkte wie die Mess-Kontaktstelle ausgebildet sein, die unter Bezugnahme auf Fig. 3 beschrieben ist.

Das erfindungsgemäße Konzept kann auch bei Ein- und Ausgängen/Schnittstellen des extrakorporalen Blutkreislaufes Verwendung finden.

## Patentansprüche

1. Schaltungsanordnung zur Schutzleiteranbindung an mindestens zwei flüssigkeitsführende Leitungen (11, 12), wobei die Schaltungsanordnung eine Mehrzahl von Schutz-Vorrichtungen (16A, 16B; 17A, 17B) zur elektrischen Kontaktierung aufweist, die derart ausgebildet sind, dass mit jeder Schutz-Vorrichtung zur elektrischen Kontaktierung eine elektrische Verbindung zu einer in der Leitung (11, 12) befindlichen Flüssigkeit herstellbar ist,
**dadurch gekennzeichnet, dass**
jeder flüssigkeitsführenden Leitung (11, 12) mindestens zwei Schutz-Vorrichtungen (16A, 16B; 17A, 17B) zur elektrischen Kontaktierung zugeordnet sind, und
die mindestens zwei Schutz-Vorrichtungen (16A, 16B) zur elektrischen Kontaktierung einer flüssigkeitsführenden Leitung (11) jeweils mit einer anderen Schutz-Vorrichtung (17A, 17B) zur elektrischen Kontaktierung einer anderen flüssigkeitsführenden Leitung (12) elektrisch verbunden sind.

2. Schaltungsanordnung nach Anspruch 1, **dadurch gekennzeichnet, dass** von den mindestens zwei Schutz-Vorrichtungen (16A, 16B; 17A, 17B) zur elektrischen Kontaktierung, die jeder flüssigkeitsführenden Leitung (11, 12) zugeordnet sind, mindestens eine Schutz-Vorrichtung zur elektrischen Kontaktierung einem im Inneren des Gehäuses (1) des medizintechnischen Gerätes liegenden Leitungsabschnitt (11A, 12A) der flüssigkeitsführenden Leitung (11, 12) und mindestens eine Schutz-Vorrichtung zur elektrischen Kontaktierung einem zumindest teilweise außerhalb des Gehäuses liegenden Leitungsabschnitt (11B, 12B) der flüssigkeitsführenden Leitung (11, 12) zugeordnet sind.

3. Schaltungsanordnung nach Anspruch 2, **dadurch gekennzeichnet, dass** eine Schutz-Vorrichtung (16A, 17A) zur elektrischen Kontaktierung einer flüssigkeitsführenden Leitung (11, 12), die dem im Inneren des Gehäuses liegenden Leitungsabschnitt der einen flüssigkeitsführenden Leitung zugeordnet ist, mit einer Schutz-Vorrichtung (16B, 17B) zur elektrischen Kontaktierung einer anderen flüssigkeitsführenden Leitung (11, 12), die dem zumindest teilweise außerhalb des Gehäuses liegenden Leitungsabschnitt der anderen flüssigkeitsführenden Leitung zugeordnet ist, elektrisch verbunden ist.

4. Schaltungsanordnung nach Anspruch 3, **dadurch gekennzeichnet, dass** die dem im Inneren des Gehäuses liegenden Leitungsabschnitt der flüssigkeitsführenden Leitung zugeordneten Schutz-Vorrichtungen (16A, 17A) zur elektrischen Kontaktierung jeweils mit einer elektrischen Verbindungsleitung (24, 25) an einen Schutzleiter oder einen Potentialausgleich (15) angeschlossen sind.

5. Medizintechnisches Gerät mit einem Gehäuse und mindestens zwei flüssigkeitsführende Leitungen (11, 12), **dadurch gekennzeichnet, dass** das medizintechnische Gerät eine Schaltungsanordnung nach einem der Ansprüche 1 bis 4 aufweist.

6. Medizintechnisches Gerät nach Anspruch 5, **dadurch gekennzeichnet, dass** die mindestens zwei flüssigkeitsführenden Leitungen (11, 12) von dem Inneren des Gehäuses nach Außen geführt sind.

7. Medizintechnisches Gerät nach Anspruch 5 oder 6, **dadurch gekennzeichnet, dass** die mindestens zwei flüssigkeitsführenden Leitungen (11, 12) Teil eines Flüssigkeitssystems (9) des medizintechnischen Gerätes sind.

8. Medizintechnisches Gerät nach einem der Ansprüche 5 bis 7, **dadurch gekennzeichnet, dass** sich in den mindestens zwei flüssigkeitsführenden Leitungen (11, 12) eine medizinische Flüssigkeit, insbesondere Dialysat, befindet.

9. Medizintechnisches Gerät nach einem der Ansprüche 5 bis 8, **dadurch gekennzeichnet, dass** das medizintechnische Gerät eine erste flüssigkeitsführende Leitung (11) und eine zweite flüssigkeitsführende Leitung (12) aufweist, wobei die beiden flüssigkeitsführenden Leitungen einen Flüssigkeitskreis (9) bilden.

10. Medizintechnisches Gerät nach einem der Ansprüche 5 bis 9, **dadurch gekennzeichnet, dass** das medizintechnische Gerät ein Blutbehandlungsgerät, insbesondere ein Dialysegerät ist.

11. Medizintechnisches Gerät nach einem der Ansprüche 5 bis 10, **dadurch gekennzeichnet, dass** das Gehäuse (1) aus einem elektrisch leitenden Material besteht, wobei das Gehäuse elektrisch mit dem Schutzleiter oder Potentialausgleich (15) verbunden ist.

12. Medizintechnisches Gerät nach einem der Ansprüche 5 bis 11, **dadurch gekennzeichnet, dass** das Gehäuse (1) aus einem nicht-elektrisch leitenden Material besteht, wobei eine von Außen zugängliche elektrische Mess-Kontaktstelle (27) am Gehäuse elektrisch mit dem Schutzleiter oder Potentialausgleich (15) verbunden ist.

13. Medizintechnisches Gerät nach einem der Ansprüche 5 bis 12, **dadurch gekennzeichnet, dass** der Schutzleiter oder Potentialausgleich (15) mit dem Schutzleitersystem einer festen Elektroinstallation verbunden ist.

14. Medizintechnisches Gerät nach einem der Ansprüche 1 bis 13, **dadurch gekennzeichnet, dass** mindestens eine Schutz-Vorrichtung (16A, 16B; 17A, 17B) als eine Gehäusedurchführung ausgebildet ist.

15. Medizintechnisches Gerät nach Anspruch 14, **dadurch gekennzeichnet, dass** die mindestens eine als Gehäusedurchführung ausgebildete Schutz-Vorrichtung (16B', 17B') gegenüber einem Gehäuse (1) aus einem elektrisch leitenden Material elektrisch isoliert ist.

16. Verfahren zur Überprüfung einer Schutzleiteranbindung an mindestens zwei flüssigkeitsführende Leitungen (11, 12), die von dem Inneren eines Gehäuses (1) eines medizintechnischen Gerätes nach Außen geführt sind, mit einer Schaltungsanordnung nach Anspruch 4, **dadurch gekennzeichnet, dass** der elektrische Widerstand zwischen den Schutz-Vorrichtungen (16B, 17B) zur elektrischen Kontaktierung, die dem zumindest teilweise außerhalb des Gehäuses liegenden Leitungsabschnitten (11B, 12B) der flüssigkeitsführenden Leitungen zugeordnet sind, und dem Schutzleiter oder Potentialausgleich (15) gemessen wird.

17. Verfahren nach Anspruch 16, **dadurch gekennzeichnet, dass** der Schutzleiter oder Potentialausgleich (15) elektrisch mit einem Gehäuse (1) aus einem elektrisch leitenden Material verbunden wird, wobei der elektrische Widerstand zwischen den Schutz-Vorrichtungen (16B, 17B) zur elektrischen Kontaktierung, die dem zumindest teilweise außerhalb des Gehäuses liegenden Leitungsabschnitten (11B, 12B) der flüssigkeitsführenden Leitungen (11, 12) zugeordnet sind, und dem Gehäuse (1) gemessen wird.

18. Verfahren nach Anspruch 16, **dadurch gekennzeichnet, dass** der Schutzleiter oder Potentialausgleich (15) elektrisch mit einer von Außen zugänglichen elektrischen Mess-Kontaktstelle (27) an einem Gehäuse (1) aus einem elektrisch nicht-leitenden Material verbunden wird, wobei der elektrische Widerstand zwischen den Schutz-Vorrichtungen (16B, 17B) zur elektrischen Kontaktierung, die dem zumindest teilweise außerhalb des Gehäuses liegenden Leitungsabschnitten (11B, 12B) der flüssigkeitsführenden Leitungen (11, 12) zugeordnet sind, und der Mess-Kontaktstelle (27) gemessen wird.

## Claims

1. Circuit for connecting a protective-conductor to at least two liquid-conveying lines (11, 12), the circuit comprising a plurality of protection apparatuses (16A, 16B; 17A, 17B) for electrical contacting which are designed such that an electrical connection to a liquid in the line (11, 12) can be produced using each protection apparatus for electrical contacting,
**characterised in that**
at least two protection apparatuses (16A, 16B; 17A, 17B) for electrical contacting are assigned to each liquid-conveying line (11, 12), and
the at least two protection apparatuses (16A, 16B) for electrically contacting a liquid-conveying line (11) are each electrically connected to another protection apparatus (17A, 17B) for electrically contacting another liquid-conveying line (12).

2. Circuit according to claim 1, **characterised in that**, out of the at least two protection apparatuses (16A, 16B; 17A, 17B) for electrical contacting assigned to each liquid-conveying line (11, 12), at least one protection apparatus for electrical contacting is assigned to a line portion (11A, 12A) of the liquid-conveying line (11, 12) that is inside the housing (1) and at least one protection apparatus for electrical contacting is assigned to a line portion (11B, 12B) of the liquid-conveying line (11, 12) that is outside the housing, at least in part.

3. Circuit according to claim 2, **characterised in that** a protection apparatus (16A, 17A) for electrically contacting a liquid-conveying line (11, 12) that is assigned to the line portion of one of the liquid-conveying lines that is inside the housing is electrically connected to a protection apparatus (16B, 17B) for electrically contacting another liquid-conveying line (11, 12) that is assigned to the line portion of the other liquid-conveying line that is outside the housing, at least in part.

4. Circuit according to claim 3, **characterised in that** the protection apparatuses (16A, 17A) assigned to the line portion of the liquid-conveying line that is inside the housing are each connected to a protective conductor or an equipotential bonding system (15) by means of an electrical connecting line (24, 25).

5. Medico-technical device comprising a housing and at least two liquid-conveying lines (11, 12), **characterised in that** the medico-technical device is a circuit according to any of claims 1 to 4.

6. Medico-technical device according to claim 5, **characterised in that** the at least two liquid-conveying lines (11, 12) are guided outwards from the interior of the housing.

7. Medico-technical device according to either claim 5 or claim 6, **characterised in that** the at least two liquid-conveying lines (11, 12) are part of a liquid system (9) of the medico-technical device.

8. Medico-technical device according to any of claims 5 to 7, **characterised in that** a medical liquid, in particular dialysate, is located in the at least two liquid-conveying lines (11, 12).

9. Medico-technical device according to any of claims 5 to 8, **characterised in that** the medico-technical device comprises a first liquid-conveying line (11) and a second liquid-conveying line (12), the two liquid-conveying lines forming a liquid circuit (9).

10. Medico-technical device according to any of claims 5 bis 9, **characterised in that** the medico-technical device is a blood treatment device, in particular a dialysis device.

11. Medico-technical device according to any of claims 5 to 10, **characterised in that** the housing (1) consists of an electrically conductive material, the housing being electrically connected to the protective conductor or equipotential bonding system (15).

12. Medico-technical device according to any of claims 5 to 11, **characterised in that** the housing (1) consists of an electrically non-conductive material, an electrical measurement contact point (27) on the housing being electrically connected to the protective conductor or equipotential bonding system (15), which point is accessible from the outside.

13. Medico-technical device according to any of claims 5 to 12, **characterised in that** the protective conductor or equipotential bonding system (15) is connected to the protective conductor system of a fixed electrical installation.

14. Medico-technical device according to any of claims 1 to 13, **characterised in that** at least one protection apparatus (16A, 16B; 17A, 17B) is designed as a housing feedthrough.

15. Medico-technical device according to claim 14, **characterised in that** the at least one protection apparatus (16B', 17B') designed as a housing feedthrough is electrically insulated with respect to a housing (1) made of an electrically conductive material.

16. Method for testing a protective-conductor connection to at least two liquid-conveying lines (11, 12) that are guided outwards from the interior of a housing of a medico-technical housing (1) by means of a circuit according to claim 4, **characterised in that** the electrical resistance between the protection apparatuses (16B, 17B) for electrical contacting that are assigned to the line portions (11B, 12B) of the liquid-conveying lines that are outside the housing, at least in part, and the protective conductor or equipotential bonding system (15) is measured.

17. Method according to claim 16, **characterised in that** the protective conductor or equipotential bonding system (15) is electrically connected to a housing (1) made of an electrically conductive material, the electrical resistance between the protection apparatuses (16B, 17B) for electrical contacting that are assigned to the line portions (11B, 12B) of the liquid-conveying lines (11, 12) that are outside the housing, at least in part, and the housing (1) being measured.

18. Method according to claim 16, **characterised in that** the protective conductor or equipotential bonding system (15) is electrically connected to an electrical measurement contact point (27), which is accessible from the outside, on a housing (1) made of an electrically non-conductive material, the electrical resistance between the protection apparatuses (16B, 17B) for electrical contacting that are assigned to the line portions (11B, 12B) of the liquid-conveying lines (11, 12) that are outside the housing, at least in part, and the measurement contact point (27) being measured.

## Revendications

1. Ensemble circuit pour la connexion par conducteur de protection à au moins deux conduites à circulation de liquide (11, 12), l'ensemble circuit comportant une pluralité de dispositifs de protection (16A, 16B ; 17A, 17B) de mise en contact électrique, qui sont conçus de telle sorte que chaque dispositif de protection de mise en contact électrique permet d'établir une connexion électrique à un liquide se trouvant dans la conduite (11, 12),
**caractérisé en ce que**
au moins deux dispositifs de protection (16A, 16B ; 17A, 17B) de mise en contact électrique sont associés à chaque conduite à circulation de liquide (11, 12), et
lesdits au moins deux dispositifs de protection (16A, 16B) de mise en contact électrique d'une conduite à circulation de liquide (11) sont chacun connectés électriquement à un autre dispositif de protection (17A, 17B) de mise en contact électrique d'une autre conduite à circulation de liquide (12).

2. Ensemble circuit selon la revendication 1,
**caractérisé en ce que** parmi lesdits au moins deux dispositifs de protection (16A, 16B ; 17A, 17B) de mise en contact électrique, qui sont associés à chaque conduite à circulation de fluide (11, 12), au moins un dispositif de protection de mise en contact électrique est associé à une section de conduite (11A, 12A) de la conduite à circulation de liquide (11, 12) qui est située à l'intérieur du boîtier (1) de l'appareil médical, et au moins un dispositif de protection de mise en contact électrique est associé à une section de conduite (11B, 12B) de la conduite à circulation de liquide (11, 12) qui est située au moins en partie à l'extérieur du boîtier.

3. Ensemble circuit selon la revendication 2,
**caractérisé en ce qu'**un dispositif de protection (16A, 17A) de mise en contact électrique d'une conduite à circulation de liquide (11, 12), qui est associé à la section de conduite de la conduite à circulation de liquide située à l'intérieur du boîtier, est connecté électriquement à un dispositif de protection (16B, 17B) de mise en contact électrique d'une autre conduite à circulation de liquide (11, 12), qui est associé à la section de conduite de l'autre conduite à circulation de liquide située au moins en partie à l'extérieur du boîtier.

4. Ensemble circuit selon la revendication 3,
**caractérisé en ce que** les dispositifs de protection (16A, 17A) de mise en contact électrique, associés à la section de conduite de la conduite à circulation de liquide située à l'intérieur du boîtier, sont chacun raccordés à un conducteur de protection ou à une liaison équipotentielle (15) par l'intermédiaire d'une ligne de connexion électrique (24, 25).

5. Appareil médical comprenant un boîtier et au moins deux conduites à circulation de liquide (11, 12),
**caractérisé en ce que** l'appareil médical comprend un ensemble circuit selon l'une des revendications 1 à 4.

6. Appareil médical selon la revendication 5,
**caractérisé en ce que** lesdites au moins deux conduites à circulation de liquide (11, 12) sont menées de l'intérieur du boîtier vers l'extérieur.

7. Appareil médical selon la revendication 5 ou 6,
**caractérisé en ce que** lesdites au moins deux conduites à circulation de liquide (11, 12) font partie d'un système à liquide (9) de l'appareil médical.

8. Appareil médical selon l'une des revendications 5 à 7,
**caractérisé en ce que** lesdites au moins deux conduites à circulation de liquide (11, 12) contiennent un liquide médical, en particulier un dialysat.

9. Appareil médical selon l'une des revendications 5 à 8,
**caractérisé en ce que** l'appareil médical comporte une première conduite à circulation de liquide (11) et une deuxième conduite à circulation de liquide (12), les deux conduites à circulation de liquide formant un circuit de liquide (9).

10. Appareil médical selon l'une des revendications 5 à 9,
**caractérisé en ce que** l'appareil médical est un appareil de traitement du sang, en particulier un appareil de dialyse.

11. Appareil médical selon l'une des revendications 5 à 10,
**caractérisé en ce que** le boîtier (1) est constitué d'un matériau électriquement conducteur, le boîtier étant connecté électriquement au conducteur de protection ou à la liaison équipotentielle (15).

12. Appareil médical selon l'une des revendications 5 à 11,
**caractérisé en ce que** le boîtier (1) est constitué d'un matériau non conducteur d'électricité, un point de contact de mesure électrique (27) accessible de l'extérieur et situé sur le boîtier étant connecté électriquement au conducteur de protection ou à la liaison équipotentielle (15).

13. Appareil médical selon l'une des revendications 5 à 12,
**caractérisé en ce que** le conducteur de protection ou la liaison équipotentielle (15) est connecté au système de conducteurs de protection d'une installation électrique fixe.

14. Appareil médical selon l'une des revendications 1 à 13,
**caractérisé en ce qu'**au moins un dispositif de protection (16A, 16B ; 17A, 17B) est conçu comme un passage de boîtier.

15. Appareil médical selon la revendication 14,
**caractérisé en ce que** ledit au moins un dispositif de protection (16B', 17B') conçu comme un passage de boîtier est isolé électriquement par rapport à un boîtier (1) en matériau électriquement conducteur.

16. Procédé pour contrôler une connexion par conducteur de protection à au moins deux conduites à circulation de liquide (11, 12) qui sont menées vers l'extérieur depuis l'intérieur d'un boîtier (1) d'un appareil médical, comprenant un ensemble circuit selon la revendication 4,
**caractérisé en ce que** l'on mesure la résistance électrique entre les dispositifs de protection (16B, 17B) de mise en contact électrique, associés aux sections de conduite (11B, 12B) des conduites à circulation de liquide situées au moins en partie à l'extérieur du boîtier, et le conducteur de protection ou la liaison équipotentielle (15).

17. Procédé selon la revendication 16,
**caractérisé en ce que** l'on relie électriquement le conducteur de protection ou la liaison équipotentielle (15) à un boîtier (1) en un matériau électriquement conducteur, et on mesure la résistance électrique entre les dispositifs de protection (16B, 17B) de mise en contact électrique, associés aux sections de conduite (11B, 12B) des conduites à circulation de liquide (11, 12) situées au moins en partie à l'extérieur du boîtier, et le boîtier (1).

18. Procédé selon la revendication 16,
**caractérisé en ce que** l'on relie électriquement le conducteur de protection ou la liaison équipotentielle (15) à un point de contact de mesure électrique (27) accessible de l'extérieur et situé sur un boîtier (1) en matériau non conducteur d'électricité, et on mesure la résistance électrique entre les dispositifs de protection (16B, 17B) de mise en contact électrique, associés aux sections de conduite (11B, 12B) des conduites à circulation de liquide (11, 12) situées au moins en partie à l'extérieur du boîtier, et le point de contact de mesure (27).
